# EUROPEAN PATENT APPLICATION

(11) **EP 4 331 487 A1**
(43) Date of publication of application: **06.03.2024**
(21) Application number: 23193712.9
(22) Date of filing: 28.08.2023
(51) Int. Cl.: A61B 5/22, A61B 5/00, A63B 23/20

(54) **CALIBRATION FOR INTRA-BODY PRESSURE SENSOR MEASUREMENTS BASED ON ORIENTATION THEREOF**

(30) Priority: 30.08.2022 EP 22398019; 29.03.2023 EP 23398005
(71) Applicant: Sword Health, S.A., 4100-467 Porto (PT)
(72) Inventor: Marques Gabriel, Ivo Emanuel, 4100-467 Porto (PT); Ramos de Magalhães, Ivo Jorge, 4100-467 Porto (PT); Coelho Alves, José Carlos, 4100-467 Porto (PT); Moutinho Colunas, Márcio Filipe, 4100-467 Porto (PT); Ferreira Flores Martins, Cristina, 4100-467 Porto (PT); Oliveira Santos, Pedro Henrique, 4100-467 Porto (PT); Dias Andrade, João Paulo, 4100-467 Porto (PT); Cardeano Pedrosa e Milheiro Maia, Marta Maria, 4100-467 Porto (PT); Ferro Bento, Virgílio António, 4100-467 Porto (PT)
(74) Representative: Balder IP Law, S.L.

(57) **Abstract**

Disclosed is a computer-implemented method. The method comprises processing, by at least one computing device, exercise measurements taken by a pressure sensor positioned adjacent to a pelvic floor muscle (PFM) of a person at least during performance of a physical exercise, wherein the exercise measurements are indicative of a pressure exerted on the pressure sensor and an orientation of the sensor. The method also comprises calibrating, by the at least one computing device, the pressure of the processed exercise measurements at least based on the orientation of the processed exercise measurements.

## Description

### CROSS-REFERENCE

This application claims priority to European Patent Application No. 22398019.4, filed August 30, 2022, wherein is entirely herein incorporated by reference.

### BACKGROUND

Many illnesses or injuries can be prevented from happening and recovered from by adequate physical exercising. In fact, recovery by way of physical rehabilitation speeds up the recovery process and improves the physical condition of the subject.

The pelvic floor muscle, PFM, plays an important role in possible illnesses and injuries in the lower abdominal area. Like with any other part of the body, exercising the PFM may result in an injury and/or non-improvement of the condition of the subject when not properly done, making supervision by a physical therapist or a personal trainer important. Therapists and trainers observe the subject during the exercising, determine if the subject does not properly execute the exercises, and provide guidance for the correct execution.

Intra-body pressure sensors may be suitable for digitally supervised PFM exercising. But getting data from a pressure sensor may be difficult because the sensor frequently has considerable freedom for orientation thereof within the body cavity. Pressure the muscles of the subject may exert on the sensor may vary depending on the orientation of the sensor. Thus, the freedom of orientation may compromise the accuracy of collected pressure measurements, making effective supervision more difficult.

### SUMMARY

In an aspect, a computer-implemented method is disclosed. The method comprises processing, by at least one computing device, first calibration measurements taken while a person has a first set of predetermined calibration positions such that, in each position of the first set, a pelvic floor muscle, PFM, of the person is in a first PFM state, the first calibration measurements being taken by a pressure sensor arranged inside the person adjacent to the PFM, the pressure sensor at least comprising an accelerometer, and the first calibration measurements being representative of both: pressure exerted on the pressure sensor by muscles of the person and an orientation of the pressure sensor each position of the first set. The method also comprises processing, by the at least one computing device, second calibration measurements taken while the person has a second set of predetermined calibration positions such that, in each position of the second set, the PFM is in a second PFM state, the second calibration measurements being taken by the pressure sensor arranged inside the person adjacent to the PFM, and the second calibration measurements being representative of both: the pressure exerted on the pressure sensor by the muscles of the person and the orientation of the pressure sensor in each position of the second set. The method also comprises providing, by the at least one computing device, at least one calibration model that relates the pressure exerted on the pressure sensor by the muscles of the person to the orientations of the pressure sensor based on the processed first and second calibration measurements.

In an aspect, a computer-implemented method is disclosed. The method may comprise processing, by at least one computing device, exercise measurements taken by a pressure sensor positioned adjacent to a pelvic floor muscle (PFM) of a person at least during performance of a physical exercise, wherein the exercise measurements are indicative of a pressure exerted on the pressure sensor and an orientation of the sensor. The method may also comprise calibrating, by the at least one computing device, the pressure of the processed exercise measurements at least based on the orientation of the processed exercise measurements.

In some embodiments, the pressure of the processed exercise measurements are calibrated using at least one calibration model that comprises a relationship between the pressure and the orientation of the pressure sensor.

In some embodiments, the at least one calibration model is based on one or more of a linear regression, quadratic regression, logistic regression or machine learning-based algorithm.

In some embodiments, the at least one calibration model comprises at least one multi-personal calibration model that relates pressure measurements to orientations of the pressure sensor based on calibration measurements collected for predetermined calibration positions for a plurality of calibration subjects.

In some embodiments, the computer-implemented method further comprises: receiving or providing the at least one multi-personal calibration model; and modifying the at least one multi-personal calibration model based on one or more factors indicative of a maximum pressure of the PFM in different states.

In some embodiments, the at least one multi-personal calibration model is further calibrated based on additional sensor measurements collected from the person.

In some embodiments, the physical exercise comprises at least one of lengthening and contracting of the PFM.

In some embodiments, the computer-implemented method further comprises: determining, by the at least one computing device, whether the physical exercise has been executed based on whether the calibrated pressure of the processed exercise measurements fulfill at least one exercise requirement; and providing, by the at least one computing device, one or more instructions or signals indicative of whether the physical exercise has been executed or to repeat or continue the physical exercise.

In some embodiments, the pressure of the processed exercise measurements is calibrated when an orientation of the processed exercise measurements is within a predetermined orientation range.

In some embodiments, calibrating the pressure of the processed exercise measurements comprises providing one or more calibration values associated with the at least one calibration model. Calibrating the pressure also comprises comparing the pressure of the processed exercise measurements with the one or more calibration values to determine a level with which the PFM has been in one or more of a plurality of different PFM states.

In some embodiments, the computer-implemented method further comprises: processing, by at least one computing device, calibration measurements taken during one or more calibration positions corresponding to one or more different PFM states; and providing, by the at least one computing device, the at least one calibration model based at least on the processed first and second calibration measurements.

In some embodiments, the calibration measurements comprise first and second calibration measurements comprising pressure and orientation measurements taken by the pressure sensor arranged adjacent to the PFM of the person during first and second calibration positions, respectively.

In some embodiments, the computer-implemented method further comprises: processing, by the at least one computing device, third calibration measurements taken during at least one first calibration position corresponding to a first PFM state. The at least one calibration model is further based at least on the processed third calibration measurements.

In some embodiments, the at least one calibration model comprises: a first calibration model for a first PFM state based on the processed first calibration measurements; and a second calibration model for the second PFM state based on the processed second calibration measurements.

In some embodiments, the computer-implemented method further comprises providing, by the at least one computing device, one or more instructions or signals indicative of each position of the one or more calibration positions.

In some embodiments, the computer-implemented method further comprises: processing, by the at least one computing device, verification measurements taken during the one or more calibration positions by at least one optical sensor positioned to capture an image or video of at least a portion of the person or at least one motion tracking device arranged on the person. The at least one calibration model is provided when the processed verification measurements are indicative of the person having been in each position of the one or more calibration positions.

In some embodiments, the at least one motion tracking device comprises a gyroscope and an accelerometer.

In some embodiments, the at least one calibration model consists of a single calibration model when the at least one computing device determines a correlation between the processed first calibration measurements and the processed second calibration measurements that exceeds a predetermined correlation threshold.

In an aspect, a system for tracking pelvic floor muscle (PFM) exercises is disclosed. The system comprises an intra-body pressure sensor adapted for introduction into a vagina or an anus of a person. The system also comprises a computing device comprising a processor operative to: process exercise measurements taken by a pressure sensor positioned adjacent to a pelvic floor muscle (PFM) of a person at least during performance of a physical exercise. The exercise measurements are indicative of a pressure exerted on the pressure sensor and an orientation of the sensor; and calibrate the pressure of the processed exercise measurements at least based on the orientation of the processed exercise measurements.

A first aspect of the disclosure relates to a method comprising: processing, by at least one computing device, first calibration measurements taken while a subject has a first set of predetermined calibration positions such that, in each position of the first set, a pelvic floor muscle, PFM, of the subject is in a first PFM state, the first calibration measurements being taken by a pressure sensor arranged inside the subject, the pressure sensor at least comprising an accelerometer, and the first calibration measurements being representative of both: pressure exerted on the pressure sensor by muscles of the subject and an orientation of the pressure sensor each position of the first set; processing, by the at least one computing device, second calibration measurements taken while the subject has a second set of predetermined calibration positions such that, in each position of the second set, the PFM is in a second PFM state, the second calibration measurements being taken by the pressure sensor arranged inside the subject, and the second calibration measurements being representative of both: the pressure exerted on the pressure sensor by the muscles of the subject and the orientation of the pressure sensor in each position of the second set; and providing, by the at least one computing device, at least one calibration model that relates the pressure exerted on the pressure sensor by the muscles of the subject to the orientations of the pressure sensor based on the processed first and second calibration measurements.

The calibration of the intra-body pressure sensor relies on calibration measurements thereof when the subject has been in several positions or postures according to the first and second sets of predetermined positions or postures. The intra-body pressure sensor measures the pressure exerted by muscles inside the vaginal cavity or the anal cavity so that the sensor is adjacent to the PFM; the pressure sensor is preferably maintained in the same position inside the vaginal cavity or the anal cavity during the calibration steps and subsequent steps, if any, in which further measurements are taken and, possibly, calibrated. Preferably, the sensor is arranged as close as possible to the PFM even if not in contact with the PFM.

Each predetermined position or posture involves one or more body members or muscles of the subject to be positioned and/or oriented in a certain manner, and/or be in certain states, e.g., the PFM being in relaxed state and/or contracted state and/or lengthened state, etc. By way of example, some predetermined positions or postures may require the subject to be standing, lying, sitting, etc., with e.g., one or both legs raised, the hip moved to one side, etc. It will be noted that any position or posture of a subject may be a predetermined position or posture within the scope of the present disclosure.

The pressure sensor usually rotates and/or moves in these positions or postures towing to the motion of the body members and muscles of the subject, regardless of the subject staying at a same location or moving to a new location within e.g., the same premises or at different premises. The orientations of the pressure sensor influence the pressure measurements for the same PFM state. The computing device(s) use the measured pressure and orientation to define the at least one calibration model that enables calibration of subsequent measurements whenever subsequent measurements are processed during a physical exercise not being part of a calibration procedure. The calibration of measurements is made, for example, by referencing those measurements to some calibration values, or by adjusting those measurements by means of the calibration values that, once applied to the measurements, convert them into a meaningful reference.

Other factors such as the anatomy of the subject or the health status of the PFM also influence the pressure measurements, but those factors are automatically taken into account as they vary the pressure values of the first and second calibration measurements.

By providing a calibration model for at least two PFM states, each state being per set of calibration measurements, monitoring and supervision of the lengthening/contracting of the PFM during rehabilitation and exercising becomes meaningful, because the level of lengthening or contracting achieved by the subject may be determined and compared with some reference values representative of the lengthening or contracting expected from the subject to improve her/his condition. Without the calibration model, depending on how the pressure sensor is oriented inside the subject, the pressure values of any measurements made during rehabilitation or exercising will not be associable with the relaxed state and/or a certain level of lengthening/contracting of the PFM, thus the rehabilitation or exercising cannot be correctly monitored or supervised.

In some embodiments, each of the first and second PFM states is a different one of: the PFM being in relaxed state, the PFM being in contracted state, and the PFM being in lengthened state.

In some embodiments, the method further comprises: processing, by the at least one computing device, third calibration measurements taken while the subject has a third set of predetermined calibration positions such that, in each position of the third set, the PFM is in a third PFM state, the third calibration measurements being taken by the pressure sensor arranged inside the subject, and the third calibration measurements being representative of both: the pressure exerted on the pressure sensor by the muscles of the subject and the orientation of the pressure sensor in each position of the third set; wherein the at least one calibration model is provided further based on the processed third calibration measurements.

In some embodiments, the first PFM state is the PFM being in relaxed state, the second PFM state is the PFM being in contracted state, and the third PFM state is the PFM being in lengthened state.

Some PFM exercises involve contraction, lengthening and relaxation of the PFM. The calibration includes all three PFM states for more accurate evaluation of the status of the PFM during the exercising.

In some embodiments, the at least one calibration model comprises a single calibration model when the at least one computing device determines existence of correlation between the processed first calibration measurements and the processed second calibration measurements with the correlation exceeding a predetermined correlation threshold.

One calibration model may suffice for the correct calibration of measurements of the intra-body pressure sensor whenever the pressure values of the calibration measurements behave similarly in the different PFM states.

When the ratio of change of measured pressure with respect to measured orientation is similar for two PFM states and, in one of the PFM states, the pressure may increase as the orientation increases and, in the other of the PFM states, the pressure may decrease as the orientation increases, the same calibration model could still be used with the additional definition of the trend in each PFM state using, for instance, a binary variable.

The computing device(s) determines the level of correlation between the sets of processed calibration measurements with any technique for computing correlation known in the art.

In embodiments in which further calibration measurements are provided for another PFM state, the single calibration model may also be possible when there is correlation between each set of processed calibration measurements.

In some embodiments, the at least one calibration model at least comprises: a first calibration model for the first PFM state based on the processed first calibration measurements; and a second calibration model for the second PFM state based on the processed second calibration measurements. In some embodiments, the at least one calibration model further comprises a third calibration model for the third PFM state based on the processed third calibration measurements.

Different calibration models are provided for each state the PFM is in. The provision of multiple calibration models increases the precision of referencing of subsequent measurements to the exercising capabilities of the PFM of the subject, and/or the precision of conversion(s) applied to subsequent measurements because the corresponding calibration values are based on the measurements of each state that the PFM is in.

In many cases, depending on the condition of the subject, the measured pressure values for the PFM in the first state require calibration values different from the calibration values needed for measured pressure values for the PFM in the second state. When the PFM may be in the third state, the calibration values for referencing or adjusting the measured pressure values for the PFM in the third state may be different from those for the first and/or second states.

The computing device(s) calibrates subsequent measurements provided during the exercising of the subject by considering the respective calibration model. To this end, and in addition to the orientation values of the measurements taken during the exercising session, the calibration model applicable to each pressure value might depend upon the physical exercise to be done by the subject as selection of the calibration model(s) may be made dependent upon what predetermined physical exercise is to be performed by the subject. In this sense, for adequate monitoring and supervision of the exercising by the subject, the at least one computing device has data indicative of the current (i.e., the one corresponding to the exercise measurements) predetermined physical exercise and whether the exercise involves contracting, relaxing and/or lengthening the PFM, thus based on that data it may select one or some calibration models over others.

In some embodiments, providing the at least one calibration model comprises: receiving at least one multi-personal calibration model that relates the pressure exerted on a respective pressure sensor by muscles of different people to orientations of the respective pressure sensor, or providing the at least one multi-personal calibration model based on calibration measurements taken, by the respective pressure sensor or a plurality of pressure sensors at least comprising the pressure sensor, for different people with each subject thereof respectively having the first set of predetermined calibration positions and the second set of predetermined calibration positions; and modifying the at least one multi-personal calibration model by applying first and second factors thereto, the first and second factors being representative of maximum and/or minimum pressure of the PFM in the first and second states, respectively, based on the processed first and second measurements.

The at least one calibration model may stem from one or more multi-personal calibration models defining a relationship between pressure values and orientations as measured by one or more intra-body pressure sensors in a set of people, e.g., three people, five people, ten people, fifty people, or even more. The at least one multi-personal calibration model is particularized to the subject who is going to exercise by adjustment of calibration values of the model with the first and second factors, which are subject-specific.

The relationships in the multi-personal calibration models can be obtained in the same manner as describe above, but with the averaging of the pressure values for same or similar orientations; in this sense, when the orientations are not exactly the same, e.g., interpolation or extrapolation can be used to quantify a pressure value for a given orientation used in the multi-personal calibration models.

By considering data from a set of people in the provision of calibration models, the at least one calibration model for the subject to exercise may feature fewer errors that could result from not correctly reproducing predetermined calibration positions and/or from erroneous measurements made by the pressure sensor. The at least one calibration model for the subject could be provided in less time because it does not have to be provided from scratch. At the same time, the calibration process undergone by the subject for the processing of the calibration measurements just requires finding the maximum and/or minimum pressure values for the different PFM states, and/or one, some or all sets of predetermined calibration positions can have the number of calibration positions reduced (but should have at least one) to the calibration position or positions that are known to yield the maximum and/or minimum pressure.

In some embodiments, the method further comprises: processing, by the at least one computing device, exercise measurements taken at least while the subject performs a predetermined physical exercise, the predetermined physical exercise at least comprising one of lengthening and contracting of the PFM, the exercise measurements being taken by the pressure sensor arranged inside the subject, and the exercise measurements being representative of both the pressure exerted on the pressure sensor by the muscles of the subject and the orientation of the pressure sensor; and calibrating, by the at least one computing device, the pressure of the processed exercise measurements using the at least one calibration model at least based on the orientation of the processed exercise measurements.

The exercise measurements correspond to the exercising of the PFM during a rehabilitation or exercising session. As the pressure values of the exercise measurements are associated with the orientation values of the pressure sensor when the measurements were taken, the at least one computing device calibrates the pressure values with the at least one calibration model having regard the orientation values of the exercise measurements and the orientation values in the at least one calibration model. As the model relates the pressure values to the orientation values, a calibration value may be derived therefrom for the concerned orientation value(s) of the exercise measurements.

In some embodiments, calibrating the pressure of the processed exercise measurements comprises providing one or more calibration values associated with each model of the at least one calibration model. In these embodiments, calibrating the pressure of the processed exercise measurements further comprises: comparing the pressure of the processed exercise measurements with the one or more calibration values to determine a level with which the PFM has been in the first state and/or the second state; or applying the one or more calibration values to the pressure of the processed exercise measurements for calibration thereof so that the level with which the PFM has been in the first state and/or the second state can be determined by processing the pressure with the one or more calibration values applied thereto.

The calibration of the pressure values involves using the provided calibration value(s) either as reference for quantification of the level, or as calibration factors that are applied to the measurements as e.g., multiplication constants to put all the measurements with a same reference scale so that they may be evaluated.

In some embodiments, the method further comprises determining, by the at least one computing device, whether the predetermined physical exercise has been executed according to predetermined exercise requirements based on whether the calibrated pressure of the processed exercise measurements fulfill one or more predetermined constraints associated with the predetermined exercise requirements.

One or more constraints define how the PFM shall behave during the exercise, e.g., how much it must lengthen, how much it must contract, what is the sequence of lengthening, contracting and/or relaxing, etc. These constraints can only be meaningfully evaluated for determination of whether the exercise has been correctly executed with calibrated pressure values since these are the ones that actually indicate the status of the PFM.

Checking fulfillment of the predetermined constraint or constraints may comprise evaluating whether the calibrated pressure value(s) are equal to, less than or greater than one or more predetermined thresholds, or are within or outside of predetermined ranges, and/or whether these occur according to a predetermined sequence.

In some embodiments, the pressure of the processed exercise measurements is calibrated when the orientation of the processed exercise measurements is within a predetermined orientation range.

The at least one calibration model may be made valid just for a range of all possible pressure sensor orientations. The range is to be configured depending on the calibration procedure itself, particularly how the pressure sensor had been oriented when taking the calibration measurements.

When the orientation of the pressure sensor in the exercise measurements is not within the range, the calibration is not effected and the at least one computing device preferably commands the provision of one or more user perceptible signals for notification of the subject that the pressure sensor is incorrectly oriented, and/or with indications on how to rotate the pressure sensor to be correctly oriented. Regarding the latter, the at least one computing device preferably has data indicative of how the pressure sensor shall be oriented for performance of the predetermined physical exercise. The correct orientation or set of orientations for the predetermined physical exercise may be manually configured according to experimental tests, or be automatically configured by the at least one computing device by deriving how the pressure sensor would ideally have to be oriented when the subject has a predetermined position associated with the physical exercise to be performed. By way of example, if the physical exercise requires the subject to be sitting, the orientation of the pressure sensor may be such that a main axis of the pressure sensor that is vertical when the sensor is upright shall form an angle of e.g., between 75° and 105° with respect to a gravity vector, which is measurable with an accelerometer of the pressure sensor.

In some embodiments, the method further comprises processing, by the at least one computing device, verification measurements taken while the subject has each set of predetermined calibration positions and, the verification measurements being taken by at least one optical sensor and/or by at least one motion tracking device arranged on the subject, each device of the at least one motion tracking device comprising a gyroscope and an accelerometer. Also, the at least one calibration model is provided when the processed verification measurements are indicative of the subject having been in each position of each set of predetermined calibration positions.

For the provision of accurate calibration model(s), the at least one computing device verifies that the subject correctly reproduces the predetermined calibration positions or postures. It may occur that the subject does not properly position herself/himself according to the set of predetermined calibration positions and, thus, the PFM is not in the expected states, which influences the pressure values of the calibration measurements.

The measurements of the at least one optical sensor are processed with digital processing techniques known in the art configured to identify joints of a subject when images or video footage capture the shape of a subject. For instance, but without limitation, digital libraries and algorithms like Google's TensorFlow Pose estimation, Google's MediaPipe Pose and BlazePose, the algorithm described in "Reconstructing 3D Human Pose from 2D Image Landmarks" by V. Ramakrishna, etc., are several known techniques for joint identification and tracking.

The measurements of the at least one motion tracking device are processed with digital processing techniques known in the art configured to identify motion, orientation and/or acceleration of different body members of the subject based on the measurements of the inertial measurement units thereof.

In some embodiments, at least some positions of the first set of predetermined calibration positions are such that the orientation of the pressure sensor, when arranged in the same position inside the subject, is different; and at least some positions of the second set of predetermined calibration positions are such that the orientation of the pressure sensor, when arranged in the same position inside the subject, is different.

Positions of each set of predetermined calibration positions result in an orientation of the pressure sensor that preferably differs from an orientation of the pressure sensor at other positions of the same set of predetermined calibration positions by more than a predetermined orientation threshold, e.g., 5°, 10°, etc. That way, the at least one calibration model has a greater range of orientation values for calibrating pressure values.

Preferably, all positions of each set of predetermined calibration positions result in a different orientation of the pressure sensor, and more preferably said different orientation differs from other orientations by more than the predetermined orientation threshold.

In some embodiments, the method further comprises commanding, by the at least one computing device, provision of one or more user perceptible signals at least representative of each position of each set of predetermined calibration positions.

The at least one computing device has data indicative of the calibration positions or postures that the subject is to have for adequate calibration of the intra-body pressure sensor. Said data can be provided in the form of user perceptible signals for the subject to reproduce the positions or postures and, thus, generate the expected data for provision of the at least one calibration model.

In some embodiments, each set of calibration measurements and/or the exercise measurements are further representative of acceleration of the pressure sensor.

In some embodiments, the method further comprises halting a calibration process (one or more of e.g., taking calibration measurements, providing the calibration measurements to the at least one computing device, processing the calibration measurements, providing the at least one calibration model, etc.) or exercising process (one or more of e.g., taking exercise measurements, providing the exercise measurements to the at least one computing device, processing the exercise measurements, calibrating the exercise measurements, determining whether the predetermined physical exercise has been executed according to predetermined exercise requirements, etc.) when, upon processing the acceleration of the measurements, the at least one computing device determines that pressure sensor has moved more than a predetermined motion threshold.

The subject introduces the pressure sensor inside her/his body, preferably according to some instructions provided to her/him in the form of user perceptible signals commanded for provision by the at least one computing device. Afterwards, the pressure sensor may move while being inside the body of the subject, which not only may cause the rotation of the sensor but also the change in position within the body. Whenever the pressure sensor moves, the pressure measurements thereof may not be the same as different muscles contact the pressure sensor. Hence, it is generally undesirable that the pressure sensor moves.

By processing the acceleration measurements, it can be estimated whether the sensor moved and how much. The predetermined motion threshold is preferably set so that, whenever the estimated motion exceeds it, it is determined that the pressure sensor has moved too much and, thus, confidence in the pressure measurements became low. In these situations, the process is preferably halted up until the subject repositions the pressure sensor inside the body, at which point the process can be resumed, for example upon receiving a command to that end manually inputted by the subject. As part of the halting, the at least one computing device may inform the user about the movement of the pressure sensor (through user presenting means such as a screen, loudspeakers, a haptic actuator in the pressure sensor, etc.) and give her/him instructions on how to reposition the pressure sensor by way of one or more user perceptible signals to that end.

In some embodiments, the method further comprises commanding activation of a haptic actuator of the pressure sensor when, upon processing the acceleration of the measurements, the at least one computing device determines that pressure sensor has moved more than a predetermined motion threshold.

To warn the subject about the sensor moving, haptic feedback can be provided to the subject. That way, the subject may stop moving that way or altogether to avoid incorrect placement of the sensor.

In some embodiments, the at least one calibration model comprises a plurality of calibration values provided with one of the following: linear regression, quadratic regression, logistic regression, or machine learning-based algorithm.

In some embodiments, the method further comprises: taking the calibration measurements by the pressure sensor while the subject has the respective set of predetermined calibration positions.

In some embodiments, the method further comprises: receiving, by the at least one computing device, each set of calibration measurements from the pressure sensor.

In some embodiments, the pressure sensor is an intravaginal sensor or an ano-rectal plug sensor.

A second aspect of the disclosure relates to a data processing device or system comprising: at least one processor adapted to perform a method according to the first aspect.

The device or system may be part of a system for tracking exercises and/or motion of a subject, with the system being configured to automatically and digitally supervise the performance of physical exercises involving the pelvic floor muscle.

The device or system may comprise at least one memory and computer program code stored therein with instructions that, when run by the at least one processor, cause the device or system to perform the steps, namely the device or system: process first and second (and possibly further) calibration measurements taken while a subject has first and second sets of predetermined calibration positions such that, in each position of one set, the PFM of the subject is in a respective PFM state, the calibration measurements being taken by a pressure sensor arranged inside the subject, the pressure sensor at least comprising an accelerometer, and the respective calibration measurements being representative of both: pressure exerted on the pressure sensor by muscles of the subject and an orientation of the pressure sensor each position of the respective set; and provide at least one calibration model that relates the pressure exerted on the pressure sensor by the muscles of the subject to the orientations of the pressure sensor based on the processed calibration measurements.

A third aspect of the disclosure relates to a device or system comprising: means adapted to execute the steps of a method according to the first aspect.

A fourth aspect of the disclosure relates to a system for tracking pelvic floor muscle exercises. The system comprises: an intra-body pressure sensor adapted for introduction into a vagina or an anus of a subject, the intra-body pressure sensor comprising an accelerometer; and a device or system according to the second aspect or a device according to the third aspect.

A fifth aspect of the disclosure relates to a computer program product that has instructions which, when executed by at least one computing device like e.g., at least one device according the second aspect or third aspect, cause the at least one computing device to carry out the steps of a method according to the first aspect.

In some embodiments, the computer program product is embodied on a non-transitory computer-readable medium or a computer-readable data carrier has the computer program product stored thereon.

A sixth aspect of the disclosure relates to a data carrier signal carrying a computer program product according to the fifth aspect.

A seventh aspect of the disclosure relates to the use of a method, device, system, computer program or data carrier signal as described in the previous aspects for physical rehabilitation of a subject.

Similar advantages as those set out with reference to the first aspect of the disclosure are likewise applicable to the remaining aspects of the disclosure. Another aspect of the present disclosure provides a non-transitory computer readable medium comprising machine executable code that, upon execution by one or more computer processors, implements any of the methods above or elsewhere herein.

Another aspect of the present disclosure provides a system comprising one or more computer processors and computer memory coupled thereto. The computer memory comprises machine executable code that, upon execution by the one or more computer processors, implements any of the methods above or elsewhere herein.

Additional aspects and advantages of the present disclosure will become readily apparent to those skilled in this art from the following detailed description, wherein only illustrative embodiments of the present disclosure are shown and described. As will be realized, the present disclosure is capable of other and different embodiments, and its several details are capable of modifications in various obvious respects, all without departing from the disclosure. Accordingly, the drawings and description are to be regarded as illustrative in nature, and not as restrictive.

### INCORPORATION BY REFERENCE

All publications, patents, and patent applications mentioned in this specification are herein incorporated by reference to the same extent as if each individual publication, patent, or patent application was specifically and individually indicated to be incorporated by reference. To the extent publications and patents or patent applications incorporated by reference contradict the disclosure contained in the specification, the specification is intended to supersede and/or take precedence over any such contradictory material.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings (also "Figure" and "FIG." herein), of which:
FIG. 1 shows a system, in accordance with some embodiments;
FIG. 2 illustrates a person in a first calibration position, in accordance with an embodiment;
FIG. 3 illustrates a person in a second calibration position, in accordance with an embodiment;
FIG. 4 shows a method, in accordance with some embodiments;
FIG. 5 illustrates how a first calibration model can be provided, in accordance with some embodiments;
FIG. 6 illustrates how a second calibration model can be provided, in accordance with some embodiments;
FIG. 7 shows a method, in accordance with some embodiments; and
**FIG. 8** shows a computer system that is programmed or otherwise configured to implement methods provided herein.

### DETAILED DESCRIPTION

While various embodiments of the invention have been shown and described herein, it will be obvious to those skilled in the art that such embodiments are provided by way of example only. Numerous variations, changes, and substitutions may occur to those skilled in the art without departing from the invention. It should be understood that various alternatives to the embodiments of the invention described herein may be employed.

Whenever the term "at least," "greater than," or "greater than or equal to" precedes the first numerical value in a series of two or more numerical values, the term "at least," "greater than" or "greater than or equal to" applies to each of the numerical values in that series of numerical values. For example, greater than or equal to 1, 2, or 3 is equivalent to greater than or equal to 1, greater than or equal to 2, or greater than or equal to 3.

Whenever the term "no more than," "less than," or "less than or equal to" precedes the first numerical value in a series of two or more numerical values, the term "no more than," "less than," or "less than or equal to" applies to each of the numerical values in that series of numerical values. For example, less than or equal to 3, 2, or 1 is equivalent to less than or equal to 3, less than or equal to 2, or less than or equal to 1.

### Overview

Disclosed is a method, in accordance with some embodiments. The method may process, by at least one computing device, one or more exercise measurements taken by a pressure sensor. A pressure sensor may be a mechanical device or instrument comprising one or more sensitive elements that may provide a signal (e.g., an electrical signal) in response to an applied pressure (e.g., during an exercise in which the pressure sensor is in contact with the subject). The pressure sensor may be arranged inside a cavity of a person (e.g., an implantable pressure sensor). The pressure sensor may be reusable or disposable. The pressure sensor may be positioned adjacent to a pelvic floor muscle (PFM) of the person, at least during live performance of a physical exercise. Live performance of the physical exercise may comprise calibration positions in which the PFM is in a PFM state, such as a relaxed state, a contracted state, or a lengthened state. The exercise measurements may be indicative of a pressure exerted on the pressure sensor and/or an orientation of the sensor. For example, the measurements may indicate pressure changes without significant orientation changes (e.g., when a person is sitting still but applying pressure on the sensor), The measurements may also indicate orientation changes that are not accompanied by pressure changes, as may occur during some changes in position.

An orientation of the sensor may comprise a degree of physical displacement from a reference, a tilt of the sensor or of a portion thereof, a degree of rotation of the sensor or of a portion thereof, or an angular displacement of the sensor or of a portion thereof when compared to a reference (e.g., a body member of a subject). The orientation may be expressed using Cartesian, polar, cylindrical, or spherical coordinates, using a set of axes (e.g., (x,y,z), (ρ, θ, z) or (r, θ, ϕ)). The orientation may also be expressed using the quaternion number system (e.g., a + b**i** + c**j** + d**k**), where a, b, c, and d are real numbers, and 1, **i, j,** and **k** are the basis vectors.

The physical exercise may comprise at least one of lengthening and contracting of the PFM. The physical exercise may be, for example, a bridge, kegel, happy baby pose, plank, squat, child's pose, diaphragmatic breathing, fire hydrant, lunge, deadlift, leg press, sit-up, crunch, hip hinge, good morning, or a combination thereof. The method may further comprise determining whether the physical exercise has been executed based at least in part on whether the calibrated pressure of the processed exercise measurements fulfill at least one exercise requirement, and then providing one or more instructions or signals indicative of whether the physical exercise has been executed or to repeat or continue the physical exercise. The pressure of the processed exercise measurements may be calibrated when an orientation of the processed exercise measurements is within a predetermined orientation range.

The method may next calibrate, by at least one computing device, the pressure of the processed exercise measurements. Calibrating the pressure of the processed exercise measurements may comprise providing one or more calibration values associated with at least one calibration model and comparing the pressure of the processed exercise measurements with the one or more calibration values to determine a level (e.g., a degree which can be quantified) with which the PFM has been in one or more of a plurality of different PFM states (e.g., a level of contraction, lengthening, or relaxation).

The at least one calibration model may comprise a single calibration model when the at least one computing device determines a correlation between the processed first calibration measurements and the processed second calibration measurements that exceeds a predetermined correlation threshold. The at least one calibration model may comprise a first calibration model for a first PFM state based at least in part on the processed first calibration measurements; and a second calibration model for the second PFM state based on the processed second calibration measurements. The calibration may be based at least in part on the orientation of the processed exercise measurements. For example, the pressure of the processed exercise measurements may be calibrated using at least one calibration model comprising a relationship between the pressure and the orientation of the pressure sensor. The calibration model may be based on one or more machine learning models, which may comprise one or more of a linear regression, quadratic regression, logistic regression, ridge regression, LASSO regression, support vector regression, random forest regression, neural networks (e.g., convolutional neural networks (CNNs) or recurrent neural networks (RNNs)), or the like.

The at least one calibration model may comprise at least one multi-personal calibration model. The multi-personal calibration model that relates pressure-measurements to orientations of the pressure sensor based on calibration measurements collected for predetermined calibration positions for a plurality of calibration subjects. The method may additionally comprise receiving or providing the at least one multi-personal calibration model, and modifying the at least one multi-personal calibration model based on one or more factors indicative of a maximum pressure of the PFM in different states. The at least one multi-personal calibration model may be further calibrated based on additional sensor measurements collected from the person.

The method may also comprise processing, by at least one computing device, calibration measurements taken during one or more calibration positions corresponding to one or more different PFM states and providing, by the at least one computing device, the at least one calibration model based at least on the processed first and second calibration measurements. The calibration measurements may comprise first and second calibration measurements comprising pressure and orientation measurements taken by the pressure sensor arranged adjacent to the PFM of the person during first and second calibration positions, respectively. The method may comprise providing, by the at least one computing device, one or more instructions or signals indicative of each position of the one or more calibration positions. The provided instructions may be text, audio, video, haptic, tactile, or image-based instructions, or may be a combination thereof.

The method may further comprise processing, by the at least one computing device, verification measurements taken during the one or more calibration positions by at least one optical sensor positioned to capture an image or video of at least a portion of the person or at least one motion tracking device arranged on the person. The at least one motion tracking device may comprise a gyroscope and/or an accelerometer. In some cases, the motion tracking may be performed by a mechanical motion capture system, a magnetic motion capture system, or a stretch sensor. The at least one calibration model may be provided when the processed verification measurements are indicative of the person having been in each position of the one or more calibration positions.

The method may further comprise processing, by the at least one computing device, third calibration measurements taken during at least one first calibration position corresponding to a first PFM state, The at least one calibration model may be further based at least on the processed third calibration measurements.

If an exercise or movement is performed incorrectly, the method may further comprise providing instructions to the subject to correct the movements that were wrongly executed. The subject may then be instructed to perform another exercise.

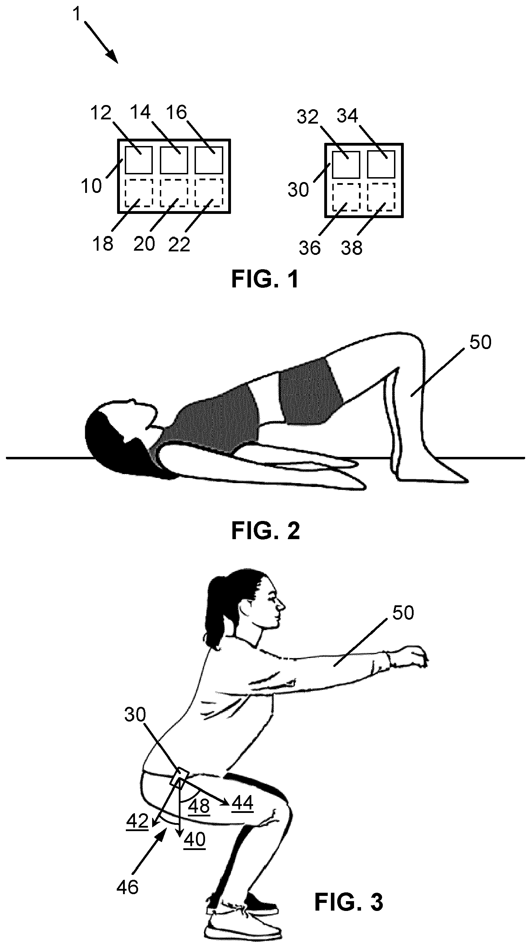

FIG. 1 shows a system 1, in accordance with some embodiments. The system 1 may include at least one computing device 10, and an intra-body pressure sensor 30.

Each computing device 10 includes at least one processor 12, at least one memory 14, and a communications module 16 at least for reception of data. The communications module 16 may likewise be adapted for transmission of data. The communications module 16, which can be for wired or wireless communications, is preferably a wireless communications module. The at least one memory 14 may store instructions and/or a computer program that, upon execution by the at least one processor 12, cause the computing device(s) 10 to supervise the execution of physical exercises by a person. The computing device(s) 10 may be e.g., a tablet, a mobile phone, a personal computer, an FPGA, an ASIC, etc.

The computing device(s) 10 may include further components, as shown with dashed lines for illustrative purposes, such as user presenting means 18 (e.g., a screen, loudspeakers, etc.), and/or user input means 20 (e.g., a touchscreen, a keyboard, etc.), and/or at least one optical sensor 22. For example, the computing device(s) 10 might guide a user through e.g., the steps for positioning herself/himself according to predetermined calibration positions or postures, which physical exercise(s) the user shall do for instance during a physical rehabilitation procedure automatically supervised by the system 1, etc.

The intra-body pressure sensor 30 is a pelvic sensor that may be, for instance, an intravaginal sensor or an ano-rectal plug sensor. The intra-body pressure sensor may be positioned adjacent to the PFM. Preferably, the pressure sensor 30 is similar or smaller in size than the size of the PFM. The pressure sensor 30 may measure a magnitude that is representative of the lengthening and contracting of the pelvic floor muscle, for example but without limitation, pressure in the pelvis or in the vagina, force applied by the pelvis, etc. The pressure sensor 30 may include a communications module 32 at least for transmission of data, the module 32 preferably being a wireless communications module. The pelvic sensor 30 may also include an accelerometer 34 and, optionally, a gyroscope 36 and/or a haptic actuator 38 for feedback to the user purposes.

Figures 2 and 3 show a person 50 in predetermined calibration positions and/or doing PFM exercises to be supervised by methods, devices and systems in accordance with embodiments.

In FIG. 2, the person 50 is lying on the floor. As part of the posture or pelvic floor exercise, the person 50 has to raise the pelvis and keep the lower legs as perpendicular to the floor as possible.

In FIG. 3, the person 50 is performing a squat. Further, in FIG. 3, an intra-body pressure sensor 30 has been shown superimposed to the person 50 for illustrative purposes only. The pressure sensor 30 is inside the vaginal cavity or the anal cavity of the person 50, as close as possible to the PFM to be as adjacent thereto as possible; for example, but without limitation, the sensor 30 may be at a distance from the PFM equal to or less than e.g., 10, 16 centimeters (4, 6 inches) from the PFM, the distance being measured with the shortest segment between the PFM and the sensor 30, so between the points thereof that are closest one to the other.

The sensor 30 may include a plurality of main axes 42, 44 (another axis going inside the page has not been illustrated). The sensor 30 can measure the direction of gravity 40 by way of its accelerometer.

An orientation of the pressure sensor 30 can be quantified, for instance, by way of an angle 46, 48 formed between one of its main axes 42, 44 and the vector corresponding to gravity 40; for the sake of clarity only, two main axes 42, 44 and two angles 46, 48 have been represented as equally possible options, but just one may be used and other possible references for measuring an angle with gravity are likewise possible. The main axis 42, 44 used for orientation measurement can be configured in advance for each particular pressure sensor 30, thus all orientation values measured by the pressure sensor 30 during the calibration process and during the exercising process may be referred to the same reference. The angle 46, 48 to be measured may, in some cases, correspond to a polar angle as defined in spherical coordinate systems.

The main axis 42, 44 used can be an axis defined by the shape of the sensor 30, e.g., a lengthwise axis, a widthwise axis, etc., or by an axis provided by an inertial measurement unit of the sensor 30. Preferably, the same axis pointing in the same direction is always used for calculating the angle with respect to gravity 40, thereby simplifying the calibration; although it is also possible to calculate the angle using the same axis but also pointing in the opposite direction if the calibration process is configured such that the smallest angle with respect to gravity 40 is to be considered every time.

It is noted that, apart from the exemplary positions and/or exercises illustrated in FIG. 2 and 3, any other position or pelvic floor exercise is possible within, and is encompassed by, the scope of the present disclosure.

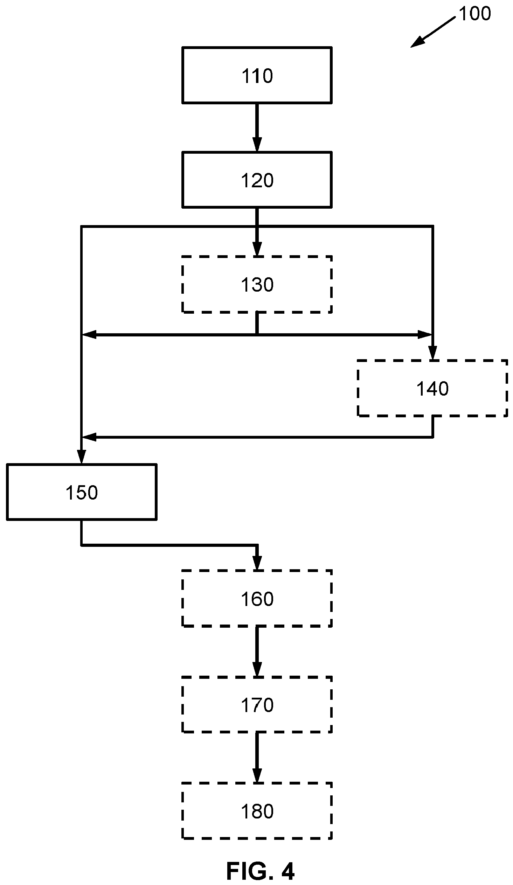

FIG. 4 shows a method 100, in accordance with some embodiments. The method may be a computer-implemented method carried out by at least one computing device.

The method 100 may include first operation 110 of processing first measurements of an intra-body pressure sensor that took the measurements while arranged inside a cavity of a person, preferably adjacent to the PFM, and the person sequentially was in different calibration positions of a first set of predetermined calibration positions in which the PFM was in a first PFM state, such as relaxed state, or contracted state, or lengthened state. The first measurements at least may include the pressure measured by the pressure sensor in each of those calibration positions, and an orientation of the pressure sensor in each of those calibration positions.

In a second operation 110, the method may include processing second measurements of the intra-body pressure sensor arranged like when providing the first measurements, but the person sequentially was in different calibration positions of a second set of predetermined calibration positions in which the PFM was in a second PFM state different from the first PFM state, such as relaxed state, or contracted state, or lengthened state. The second measurements may include the same type of data that the first measurements include.

The method 100 may also include, in some cases (as illustrated with dashed blocks), an operation of processing 130 third measurements of the intra-body pressure sensor arranged like when providing the first and second measurements, but the person sequentially was in different calibration positions of a third set of predetermined calibration positions in which the PFM was in a third PFM state different from the first PFM state and the second PFM, thus the last one among the relaxed state, the contracted state, and the lengthened state. The third measurements include the same type of data that the first and second measurements include.

The first, second and third measurements are provided to a computing device (or devices) that processes 110, 120, 130 them via a wireless or wired communications channel. The measurements may be provided to the computing device as soon as they are taken, or intermittently thereby providing them every time some measurements have been taken, or after all the measurements have been taken.

For a more accurate calibration, the person may attempt forcing a maximum PFM state in each one of the calibration positions. So: when the PFM state to be in is lengthening of the PFM in some calibration positions, the person may attempt to maximally lengthen the PFM; when the PFM state to be in is contracting of the PFM in some calibration positions, the person may attempt to maximally contract the PFM; and when the PFM state to be in is relaxing of the PFM in some calibration positions, the person may attempt to maximally relax the PFM to minimize any possible lengthening or contracting thereof.

The method 100 may also include, in some embodiments, an operation 140 of processing further measurements (e.g., third measurements, fourth measurements, etc.) of at least one optical sensor and/or by at least one motion tracking device arranged on the person taken when the person sequentially was in different calibration positions of the first, second and, optionally, third set of predetermined calibration positions. The processing 140 may limit advancing to a calibration model provision 150 step depending on the outcome of the processing 140. Particularly, the computing device evaluates the further measurements to check if the person indeed was in each position of each set of predetermined calibration positions: when the device determines that the person was not in one or more calibration positions, it may request the user to at least repeat said one or more calibration positions until the device determines that the person was in all of them; so with a positive determination of reproduction of the calibration positions, the method 100 advances to the next step. It is noted that even though the processing 140 step is shown after the processing 110, 120, 130 steps, it can take place simultaneously with those processing 110, 120, 130 steps or several times after each of the processing 110, 120, 130 steps.

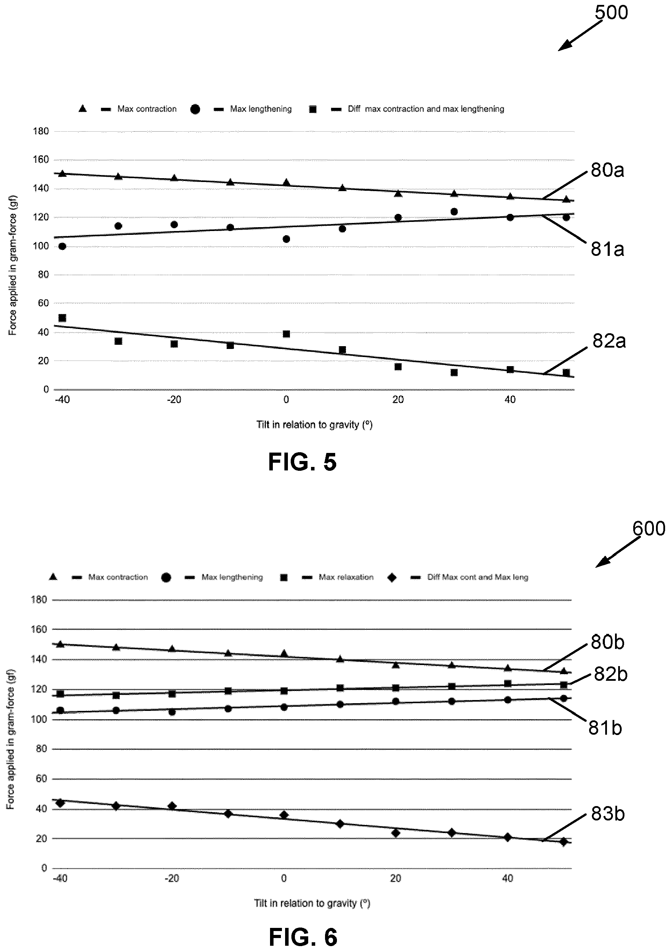

The method 100 may also include an operation 150 of providing at least one calibration model that relates the pressure exerted on the pressure sensor by the muscles of the person to the orientations of the pressure sensor based on the processed 110, 120, 130 calibration measurements, thereby making possible to calibrate pressure values of intra-body pressure sensors. Some examples of calibration model provision are shown in Figures 5 and 6.

The method 100 also includes, in some embodiments, an operation 160 of processing further measurements (e.g., third measurements, fourth measurements, fifth measurements, etc.) of the intra-body pressure sensor arranged like when providing the previous measurements, but while the person performed a predetermined physical exercise involving the lengthening and/or contracting of the PFM. These measurements may include the same type of data that the previous measurements of the intra-body pressure sensor include. Also, these measurements may be provided to the computing device via a wireless or wired communications channel. The measurements may be provided to the computing device as soon as they are taken, or intermittently (e.g., when some but not all of the measurements have been taken), or after all the measurements have been taken.

The method 100 may also include, in some embodiments, an operation 170 of calibrating pressure values of the processed 160 measurements related to the physical exercise using the at least one calibration model provided 150. For that, the orientations of the processed 160 measurements may be at least considered for using the at least one calibration model, for instance for referencing the pressure values with calibration values, or for applying calibration factors that modify the pressure values.

The method 100 may also include, in some cases, an operation 180 of determining whether the predetermined physical exercise has been executed according to predetermined exercise requirements. For that, the calibrated 170 pressure values are to fulfill one or more predetermined constraints associated with the predetermined exercise requirements. The determination 180 of correct execution may include or be combined with the processes, devices and systems for monitoring and correcting pelvic floor exercises disclosed in commonly owned European patent application no. 22398018.6, which is hereby incorporated by reference in its entirety.

FIG. 5 illustrates how a calibration model 500 can be provided in accordance with embodiments. A graph shows processed first calibration measurements with triangle markers, processed second calibration measurements with circle markers, and a difference between pressure values of the processed first and second calibration measurements with square markers. The horizontal axis corresponds to an orientation of the pressure sensor, which may be the tilt it has with respect to the gravity vector, and the vertical axis corresponds to pressure values, which in this case is quantified with grams force.

In this example, the first PFM state corresponding to a first set of predetermined calibration positions is the PFM being in contracted state, and the second PFM state corresponding to a second set of predetermined calibration positions is the PFM in lengthened state.

Upon processing the pressure and orientation values for each PFM state, one or more calibration models 80a, 81a, 82a can be provided. Calibration values (shown with a solid line) of the calibration models 80a, 81a, 82a may be provided, for example, with a linear regression. In other cases, a calibration model may be a quadratic regression, logistic regression, or machine learning-based algorithm. In this case, it can be appreciated that the calibration values in linear form more accurately represent the pressure values for the first PFM state than for the second PFM state, which can be more easily appreciated from the line of the difference between the two sets of pressure values.

Given the difference in behavior of the first and second measurements, a computing device may determine that there is no sufficient correlation between the two unlike in the example of FIG. 6. Accordingly, preferably first and second calibration models 80a, 81a are provided for calibration of measurements, in which case whenever measurements are to be calibrated, they are processed and calibrated according to the first and second calibration models 80a, 81a. By way of example, if measurements corresponding to contraction of the PFM are to be calibrated, they can be calibrated in one of the following manners:

Measurement referencing: The pressure values of the exercising session have orientation values associated therewith. The orientation values are checked against the first calibration model 80a; if the orientation values are outside of the calibration model 80a, in this case beyond the range of -40° to +50° (but it will be noted that, in other embodiments, other ranges are possible as well, even full 360°), it may be determined that calibration is not possible and, thus, the pressure sensor has to be rearranged inside the person. When the orientation falls within the range of the calibration model, the measured pressure value may be checked against the calibration value of the calibration model 80a to quantify how much pressure that is e.g., in percentage. For example, a measured pressure value of 80 gf for an orientation of -5° corresponds to a 56.3% (80 divided by 142, where 80 is the measured pressure value and 142 is the calibration value corresponding to a pressure value of 142 gf in the calibration model) contraction of the PFM attained by the person.

Measurement adjustment: When the orientation falls within the range of the calibration model, the measured pressure value is modified by a calibration value of the calibration model 80a to scale it. The calibration value is, for example, multiplicative. For example, for an orientation of the pressure sensor of +30° during an exercising session, the calibration value is e.g., 100/137=0,73 (where 100 is a scale of 100, and 137 is the calibration value corresponding to a pressure value of 137 gf in the calibration model); then, for a measured pressure value of 120 gf for said orientation, the calibrated pressure value is 120x0,73, which means that there has been a contraction of the PFM of 87,6%.

FIG. 6 illustrates how another calibration model 600 can be provided in accordance with embodiments. The same type of measurements has been used in this example, but further processed third calibration measurements are shown with square markers that correspond to the PFM being in relaxed state.

In this example, the linear regression for the processed first, second and third measurements more accurately represents the change of pressure value with respect to the tilt of the pressure sensor.

There is significant correlation (quantified and evaluated with respect to a predetermined correlation threshold) between the processed second and third measurements corresponding to the PFM in lengthened state (with circle markers) and in relaxed state (with square markers). That, in turn, makes possible to use just one single calibration model for the two PFM states that will provide accurate calibration. For example, the calibration model can rely on the model 81b, 82b provided by the linear regression of either the processed second measurements or the processed third measurements, and include, e.g., a scaling factor. By way of example, the calibration values for the PFM in relaxed state with the calibration model 81b stemming from the measurements of the PFM in lengthened state can be obtained as: 1,1 1xCVLS(tilt), where CVLS(tilt) is the calibration value for the PFM in lengthened state for a given tilt value.

The difference 83b of the processed measurements corresponding to the first and second PFM states, shown with diamond markers this time, also has a linear regression that accurately represents the values thereof. So, in this case, there is a greater correlation between pressure and orientation between the PFM in both states even if the contracting PFM state has a decreasing trend with the increase in tilt, and the lengthening PFM state has an increasing trend with the decrease in tilt. A single calibration model could be used for calibration of the PFM in contracted state (with triangle markers) and in lengthened state; or even for calibration of all three PFM states but with a more complex definition thereof to account for the decreasing trend of the first PFM state and the increases trend of the second and third PFM states.

In this example, preferably two or three calibration models are provided, a first calibration model 80b for the PFM in contracted state, and: a second calibration model 81b, 82b for the PFM in lengthened and relaxed states; or a second calibration model 81b for the PFM in lengthened state, and a third calibration model 82b for the PFM in relaxed state.

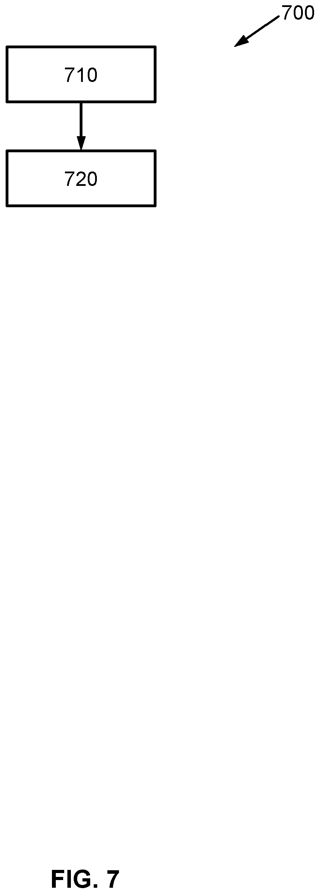

FIG. 7 shows a method 700, in accordance with some embodiments. The method may be a computer-implemented method carried out by at least one computing device.

The method may include an operation 710 of processing, by at least one computing device, exercise measurements taken by a pressure sensor. The pressure sensor may be positioned adjacent to a pelvic floor muscle (PFM) of a subject at least during performance of a physical exercise. The pressure sensor may be an intra-body pressure sensor. The pressure sensor may be arranged inside or disposed within a cavity of a subject. The exercise measurements may be indicative of a pressure exerted on the pressure sensor and/or an orientation of the sensor. The exercise measurements may comprise first measurements and second measurements. The first measurements may be taken by the pressure sensor in a sequence of calibration positions performed by the subject. During these first measurements, the PFM may be in a first PFM state, such as a relaxed state, contracted state, or lengthened state. These first measurements may include a pressure measured by the pressure sensor in at least one calibration position and/or an orientation of the pressure sensor in at least one calibration position. The second measurements may correspond to a different sequence of calibration positions. For example, at least one calibration position of the second set may differ from at least one calibration position of the first set. In some cases, the first set and the second set may include identical calibration positions, but the order in which these calibration positions is performed may be different in the first set and second set. The second set measurements may also comprise pressure measured by the pressure sensor and orientation of the pressure sensor. In some cases, the method may also include third exercise measurements ("third measurements"). The third measurements may additionally correspond to a different sequence of calibration measurements than the first and second set. At least one calibration measurement in the sequence may differ from both the first and second set. Or at least one position of at least one calibration measurement in the sequence in the third set may differ from that of the same calibration measurement in both the first and second set, even if the three sets include the same calibration positions.

The method may also include calibrating, by the at least one computing device, the pressure of the processed exercise measurements based at least in part on the orientation of the processed exercise measurements. This may comprise calibrating pressure values of the measurements (e.g., first, second, and third measurements) with at least one calibration model relating pressure exerted on the pressure sensor by muscles of the person to the orientations of the pressure sensor.

### Particular Implementations

Disclosed is a computer-implemented method comprising processing, by at least one computing device, exercise measurements taken by a pressure sensor positioned adjacent to a pelvic floor muscle (PFM) of a person at least during performance of a physical exercise. The exercise measurements may be indicative of a pressure exerted on the pressure sensor and an orientation of the sensor. The method may also include calibrating, by the at least one computing device, the pressure of the processed exercise measurements at least based on the orientation of the processed exercise measurements. The pressure of the processed exercise measurements may be calibrated using at least one calibration model that comprises a relationship between the pressure and the orientation of the pressure sensor. The at least one calibration model may be based on one or more of a linear regression, quadratic regression, logistic regression or machine learning-based algorithm. The at least one calibration model may comprise at least one multi-personal calibration model that relates pressure measurements to orientations of the pressure sensor based on calibration measurements collected for predetermined calibration positions for a plurality of calibration subjects. The method may also comprise receiving or providing the at least one multi-personal calibration model. The method may also comprise modifying the at least one multi-personal calibration model based on one or more factors indicative of a maximum pressure of the PFM in different states. The at least one multi-personal calibration model may be further calibrated based on additional sensor measurements collected from the person. The physical exercise may comprise at least one of lengthening and contracting of the PFM. The method may also comprise determining, by the at least one computing device, whether the physical exercise has been executed based on whether the calibrated pressure of the processed exercise measurements fulfill at least one exercise requirement. The method may also comprise providing, by the at least one computing device, one or more instructions or signals indicative of whether the physical exercise has been executed or to repeat or continue the physical exercise. The pressure of the processed exercise measurements may be calibrated when an orientation of the processed exercise measurements is within a predetermined orientation range. Calibrating the pressure of the processed exercise measurements may comprise providing one or more calibration values associated with the at least one calibration model, and comparing the pressure of the processed exercise measurements with the one or more calibration values to determine a level with which the PFM has been in one or more of a plurality of different PFM states. The method may also comprise processing, by at least one computing device, calibration measurements taken during one or more calibration positions corresponding to one or more different PFM states; and providing, by the at least one computing device, the at least one calibration model based at least on the processed first and second calibration measurements. The calibration measurements may also comprise first and second calibration measurements comprising pressure and orientation measurements taken by the pressure sensor arranged adjacent to the PFM of the person during first and second calibration positions, respectively. The method may also include processing, by the at least one computing device, third calibration measurements taken during at least one first calibration position corresponding to a first PFM state. The at least one calibration model may be further based at least on the processed third calibration measurements. The at least one calibration model may comprise a first calibration model for a first PFM state based on the processed first calibration measurements and a second calibration model for the second PFM state based on the processed second calibration measurements. Providing, by the at least one computing device, one or more instructions or signals indicative of each position of the one or more calibration positions. The method may also include processing, by the at least one computing device, verification measurements taken during the one or more calibration positions by at least one optical sensor positioned to capture an image or video of at least a portion of the person or at least one motion tracking device arranged on the person. The at least one calibration model is provided when the processed verification measurements are indicative of the person having been in each position of the one or more calibration positions. The at least one motion tracking device comprises a gyroscope and an accelerometer. The at least one calibration model consists of a single calibration model when the at least one computing device may determine a correlation between the processed first calibration measurements and the processed second calibration measurements that exceeds a predetermined correlation threshold. Disclosed is a system for tracking pelvic floor muscle (PFM) exercises, comprising: an intra-body pressure sensor adapted for introduction into a vagina or an anus of a person; and a computing device comprising a processor operative to: process exercise measurements taken by a pressure sensor positioned adjacent to a pelvic floor muscle (PFM) of a person at least during performance of a physical exercise. The exercise measurements are indicative of a pressure exerted on the pressure sensor and an orientation of the sensor; and calibrate the pressure of the processed exercise measurements at least based on the orientation of the processed exercise measurements.

In this text, the terms first, second, third, further, etc. have been used herein to describe several devices, elements or parameters, it will be understood that the devices, elements or parameters should not be limited by these terms since the terms are only used to distinguish one device, element or parameter from another. For example, the first calibration measurements could as well be named second calibration measurements, and the second calibration measurements could be named first calibration measurements without departing from the scope of this disclosure.

In this text, the term "includes", "comprises" and its derivations (such as "including", "comprising", etc.) should not be understood in an excluding sense, that is, these terms should not be interpreted as excluding the possibility that what is described and defined may include further elements, steps, etc. On the other hand, the disclosure is obviously not limited to the specific embodiment(s) described herein, but also encompasses any variations that may be considered by any person skilled in the art (for example, as regards the choice of materials, dimensions, components, configuration, etc.), within the general scope of the invention as defined in the claims.

### Computer systems

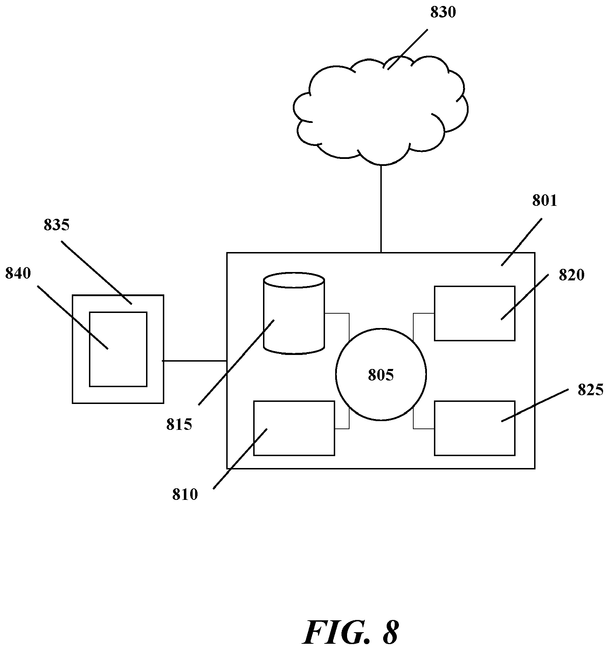

The present disclosure provides computer systems that are programmed to implement methods of the disclosure. **FIG. 8** shows a computer system 801 that is programmed or otherwise configured to process pelvic floor sensor measurements. The computer system 801 can regulate various aspects of processing pelvic floor sensor measurements of the present disclosure, such as, for example, implementing a calibration model. The computer system 801 can be an electronic device of a user or a computer system that is remotely located with respect to the electronic device. The electronic device can be a mobile electronic device.

The computer system 801 includes a central processing unit (CPU, also "processor" and "computer processor" herein) 805, which can be a single core or multi core processor, or a plurality of processors for parallel processing. The computer system 801 also includes memory or memory location 810 (e.g., random-access memory, read-only memory, flash memory), electronic storage unit 815 (e.g., hard disk), communication interface 820 (e.g., network adapter) for communicating with one or more other systems, and peripheral devices 825, such as cache, other memory, data storage and/or electronic display adapters. The memory 810, storage unit 815, interface 820 and peripheral devices 825 are in communication with the CPU 805 through a communication bus (solid lines), such as a motherboard. The storage unit 815 can be a data storage unit (or data repository) for storing data. The computer system 801 can be operatively coupled to a computer network ("network") 830 with the aid of the communication interface 820. The network 830 can be the Internet, an internet and/or extranet, or an intranet and/or extranet that is in communication with the Internet. The network 830 in some cases is a telecommunication and/or data network. The network 830 can include one or more computer servers, which can enable distributed computing, such as cloud computing. The network 830, in some cases with the aid of the computer system 801, can implement a peer-to-peer network, which may enable devices coupled to the computer system 801 to behave as a client or a server.

The CPU 805 can execute a sequence of machine-readable instructions, which can be embodied in a program or software. The instructions may be stored in a memory location, such as the memory 810. The instructions can be directed to the CPU 805, which can subsequently program or otherwise configure the CPU 805 to implement methods of the present disclosure. Examples of operations performed by the CPU 805 can include fetch, decode, execute, and writeback.

The CPU 805 can be part of a circuit, such as an integrated circuit. One or more other components of the system 801 can be included in the circuit. In some cases, the circuit is an application specific integrated circuit (ASIC).

The storage unit 815 can store files, such as drivers, libraries and saved programs. The storage unit 815 can store user data, e.g., user preferences and user programs. The computer system 801 in some cases can include one or more additional data storage units that are external to the computer system 801, such as located on a remote server that is in communication with the computer system 801 through an intranet or the Internet.

The computer system 801 can communicate with one or more remote computer systems through the network 830. For instance, the computer system 801 can communicate with a remote computer system of a user (e.g., a mobile computing device). Examples of remote computer systems include personal computers (e.g., portable PC), slate or tablet PC's (e.g., Apple^{®} iPad, Samsung^{®} Galaxy Tab), telephones, Smart phones (e.g., Apple^{®} iPhone, Android-enabled device, Blackberry^{®}), or personal digital assistants. The user can access the computer system 801 via the network 830.

Methods as described herein can be implemented by way of machine (e.g., computer processor) executable code stored on an electronic storage location of the computer system 801, such as, for example, on the memory 810 or electronic storage unit 815. The machine executable or machine readable code can be provided in the form of software. During use, the code can be executed by the processor 805. In some cases, the code can be retrieved from the storage unit 815 and stored on the memory 810 for ready access by the processor 805. In some situations, the electronic storage unit 815 can be precluded, and machine-executable instructions are stored on memory 810.

The code can be pre-compiled and configured for use with a machine having a processer adapted to execute the code, or can be compiled during runtime. The code can be supplied in a programming language that can be selected to enable the code to execute in a pre-compiled or as-compiled fashion.

Aspects of the systems and methods provided herein, such as the computer system 801, can be embodied in programming. Various aspects of the technology may be thought of as "products" or "articles of manufacture" typically in the form of machine (or processor) executable code and/or associated data that is carried on or embodied in a type of machine readable medium. Machine-executable code can be stored on an electronic storage unit, such as memory (e.g., read-only memory, random-access memory, flash memory) or a hard disk. "Storage" type media can include any or all of the tangible memory of the computers, processors or the like, or associated modules thereof, such as various semiconductor memories, tape drives, disk drives and the like, which may provide non-transitory storage at any time for the software programming. All or portions of the software may at times be communicated through the Internet or various other telecommunication networks. Such communications, for example, may enable loading of the software from one computer or processor into another, for example, from a management server or host computer into the computer platform of an application server. Thus, another type of media that may bear the software elements includes optical, electrical and electromagnetic waves, such as used across physical interfaces between local devices, through wired and optical landline networks and over various air-links. The physical elements that carry such waves, such as wired or wireless links, optical links or the like, also may be considered as media bearing the software. As used herein, unless restricted to non-transitory, tangible "storage" media, terms such as computer or machine "readable medium" refer to any medium that participates in providing instructions to a processor for execution.

Hence, a machine readable medium, such as computer-executable code, may take many forms, including but not limited to, a tangible storage medium, a carrier wave medium or physical transmission medium. Non-volatile storage media include, for example, optical or magnetic disks, such as any of the storage devices in any computer(s) or the like, such as may be used to implement the databases, etc. shown in the drawings. Volatile storage media include dynamic memory, such as main memory of such a computer platform. Tangible transmission media include coaxial cables; copper wire and fiber optics, including the wires that comprise a bus within a computer system. Carrier-wave transmission media may take the form of electric or electromagnetic signals, or acoustic or light waves such as those generated during radio frequency (RF) and infrared (IR) data communications. Common forms of computer-readable media therefore include for example: a floppy disk, a flexible disk, hard disk, magnetic tape, any other magnetic medium, a CD-ROM, DVD or DVD-ROM, any other optical medium, punch cards paper tape, any other physical storage medium with patterns of holes, a RAM, a ROM, a PROM and EPROM, a FLASH-EPROM, any other memory chip or cartridge, a carrier wave transporting data or instructions, cables or links transporting such a carrier wave, or any other medium from which a computer may read programming code and/or data. Many of these forms of computer readable media may be involved in carrying one or more sequences of one or more instructions to a processor for execution.

The computer system 801 can include or be in communication with an electronic display 835 that comprises a user interface (UI) 840 for providing, for example, providing instructions to a subject to correct a pelvic floor exercise. Examples of UI's include, without limitation, a graphical user interface (GUI) and web-based user interface.

Methods and systems of the present disclosure can be implemented by way of one or more algorithms. An algorithm can be implemented by way of software upon execution by the central processing unit 805. The algorithm can, for example, implement a calibration.

While preferred embodiments of the present invention have been shown and described herein, it will be obvious to those skilled in the art that such embodiments are provided by way of example only. It is not intended that the invention be limited by the specific examples provided within the specification. While the invention has been described with reference to the aforementioned specification, the descriptions and illustrations of the embodiments herein are not meant to be construed in a limiting sense. Numerous variations, changes, and substitutions will now occur to those skilled in the art without departing from the invention. Furthermore, it shall be understood that all aspects of the invention are not limited to the specific depictions, configurations or relative proportions set forth herein which depend upon a variety of conditions and variables. It should be understood that various alternatives to the embodiments of the invention described herein may be employed in practicing the invention. It is therefore contemplated that the invention shall also cover any such alternatives, modifications, variations or equivalents. It is intended that the following claims define the scope of the invention and that methods and structures within the scope of these claims and their equivalents be covered thereby.

Additional statements:
1. A method comprising:
   processing, by at least one computing device, first calibration measurements taken while a person has a first set of predetermined calibration positions such that, in each position of the first set, a pelvic floor muscle, PFM, of the person is in a first PFM state, the first calibration measurements being taken by a pressure sensor arranged inside the person adjacent to the PFM, the pressure sensor at least comprising an accelerometer, and the first calibration measurements being representative of both: pressure exerted on the pressure sensor by muscles of the person and an orientation of the pressure sensor each position of the first set;
   processing, by the at least one computing device, second calibration measurements taken while the person has a second set of predetermined calibration positions such that, in each position of the second set, the PFM is in a second PFM state, the second calibration measurements being taken by the pressure sensor arranged inside the person adjacent to the PFM, and the second calibration measurements being representative of both: the pressure exerted on the pressure sensor by the muscles of the person and the orientation of the pressure sensor in each position of the second set; and
   providing, by the at least one computing device, at least one calibration model that relates the pressure exerted on the pressure sensor by the muscles of the person to the orientations of the pressure sensor based on the processed first and second calibration measurements.
2. The method of statement 1, further comprising processing, by the at least one computing device, third calibration measurements taken while the person has a third set of predetermined calibration positions such that, in each position of the third set, the PFM is in a third PFM state, the third calibration measurements being taken by the pressure sensor arranged inside the person adjacent to the PFM, and the third calibration measurements being representative of both: the pressure exerted on the pressure sensor by the muscles of the person and the orientation of the pressure sensor in each position of the third set.
3. The method of any one of the preceding statements, wherein the at least one calibration model comprises a single calibration model when the at least one computing device determines existence of correlation between the processed first calibration measurements and the processed second calibration measurements with the correlation exceeding a predetermined correlation threshold.
4. The method of any one of statements 1-2, wherein the at least one calibration model at least comprises:
   a first calibration model for the first PFM state based on the processed first calibration measurements; and
   a second calibration model for the second PFM state based on the processed second calibration measurements.
5. The method of any one of the preceding statements, wherein providing the at least one calibration model comprises:
   receiving at least one multipersonal calibration model that relates the pressure exerted on a respective pressure sensor by muscles of different people to orientations of the respective pressure sensor, or providing the at least one multipersonal calibration model based on calibration measurements taken, by the respective pressure sensor or a plurality of pressure sensors at least comprising the pressure sensor, for different people with each person thereof respectively having the first set of predetermined calibration positions and the second set of predetermined calibration positions; and
   modifying the at least one multipersonal calibration model by applying first and second factors thereto, the first and second factors being representative of a maximum pressure of the PFM in the first and second states, respectively, based on the processed first and second measurements.
6. The method of any one of the preceding statements, further comprising:
   processing, by the at least one computing device, exercise measurements taken at least while the person performs a predetermined physical exercise, the predetermined physical exercise at least comprising one of lengthening and contracting of the PFM, the exercise measurements being taken by the pressure sensor arranged inside the person adjacent to the PFM, and the exercise measurements being representative of both the pressure exerted on the pressure sensor by the muscles of the person and the orientation of the pressure sensor; and
   calibrating, by the at least one computing device, the pressure of the processed exercise measurements using the at least one calibration model at least based on the orientation of the processed exercise measurements.
7. The method of statement 6, wherein calibrating the pressure of the processed exercise measurements comprises providing one or more calibration values associated with each model of the at least one calibration model, and:
   comparing the pressure of the processed exercise measurements with the one or more calibration values to determine a level with which the PFM has been in the first state and/or the second state; or
   applying the one or more calibration values to the pressure of the processed exercise measurements for calibration thereof so that it can the level with which the PFM has been in the first state and/or
   the second state can be determined by processing the pressure with the one or more calibration values applied thereto.
8. The method of any one of statements 6-7, further comprising: determining, by the at least one computing device, whether the predetermined physical exercise has been executed according to predetermined exercise requirements based on whether the calibrated pressure of the processed exercise measurements fulfill one or more predetermined constraints associated with the predetermined exercise requirements.
9. The method of any one of statements 6-8, wherein the pressure of the processed exercise measurements is calibrated when the orientation of the processed exercise measurements is within a predetermined orientation range.
10. The method of any one of the preceding statements, further comprising processing, by the at least one computing device, verification measurements taken while the person has each set of predetermined calibration positions, the verification measurements being taken by at least one optical sensor and/or by at least one motion tracking device arranged on the person, each device of the at least one motion tracking device comprising a gyroscope and an accelerometer; and wherein the at least one calibration model is provided when the processed verification measurements are indicative of the person having been in each position of each set of predetermined calibration positions.
11. The method of any one of the preceding statements, wherein at least some positions of the first set of predetermined calibration positions are such that the orientation of the pressure sensor, when arranged in the same position inside the person, is different; and at least some positions of the second set of predetermined calibration positions are such that the orientation of the pressure sensor, when arranged in the same position inside the person, is different.
12. The method of any one of the preceding statements, further comprising commanding, by the at least one computing device, provision of one or more user perceptible signals at least representative of each position of each set of predetermined calibration positions.
13. A device or system comprising: means adapted to execute the steps of a method according to any one of the preceding statements.
14. A system for tracking pelvic floor muscle exercises, comprising:
   an intra-body pressure sensor adapted for introduction into a vagina or an anus of a person, the intra-body pressure sensor comprising an accelerometer; and
   a device or system according to statement 13.
15. A computer program product that has instructions which, when the program is executed by at least one computing device, cause the at least one computing device to carry out the steps of a method according to any one of statements 1-12.

## Claims

1. A computer-implemented method comprising:
processing, by at least one computing device, exercise measurements taken by a pressure sensor positioned adjacent to a pelvic floor muscle (PFM) of a person at least during performance of a physical exercise, wherein the exercise measurements are indicative of a pressure exerted on the pressure sensor and an orientation of the sensor; and
calibrating, by the at least one computing device, the pressure of the processed exercise measurements at least based on the orientation of the processed exercise measurements.

2. The computer-implemented method of claim 1, wherein the pressure of the processed exercise measurements are calibrated using at least one calibration model that comprises a relationship between the pressure and the orientation of the pressure sensor.

3. The computer-implemented method of claim 2, wherein the at least one calibration model comprises at least one multi-personal calibration model that relates pressure measurements to orientations of the pressure sensor based on calibration measurements collected for predetermined calibration positions for a plurality of calibration subjects.

4. The computer-implemented method of claim 3, further comprising:
receiving or providing the at least one multi-personal calibration model; and
modifying the at least one multi-personal calibration model based on one or more factors indicative of a maximum pressure of the PFM in different states.

5. The computer-implemented method of any one of the previous claims, wherein the physical exercise comprises at least one of lengthening and contracting of the PFM.

6. The computer-implemented method of any one of the previous claims, further comprising:
determining, by the at least one computing device, whether the physical exercise has been executed based on whether the calibrated pressure of the processed exercise measurements fulfill at least one exercise requirement; and
providing, by the at least one computing device, one or more instructions or signals indicative of whether the physical exercise has been executed or to repeat or continue the physical exercise.

7. The method of any one of the previous claims, wherein the pressure of the processed exercise measurements is calibrated when an orientation of the processed exercise measurements is within a predetermined orientation range.

8. The computer-implemented method of claim 2 or of any one of claims 3 - 7 when directly or indirectly depending on claim 2, wherein calibrating the pressure of the processed exercise measurements comprises:
providing one or more calibration values associated with the at least one calibration model; and
comparing the pressure of the processed exercise measurements with the one or more calibration values to determine a level with which the PFM has been in one or more of a plurality of different PFM states.

9. The computer-implemented method of claim 2 or of any one of claims 3 - 8 when directly or indirectly depending on claim 2, further comprising:
processing, by at least one computing device, calibration measurements taken during one or more calibration positions corresponding to one or more different PFM states; and
providing, by the at least one computing device, the at least one calibration model based at least on processed first and second calibration measurements.

10. The computer-implemented method of any one of claims 3 - 9 when directly or indirectly depending on claim 2, wherein the calibration measurements comprise first and second calibration measurements comprising pressure and orientation measurements taken by the pressure sensor arranged adjacent to the PFM of the person during first and second calibration positions, respectively.

11. The computer-implemented method of claim 9 or 10, further comprising:
processing, by the at least one computing device, third calibration measurements taken during at least one first calibration position corresponding to a first PFM state,
wherein the at least one calibration model is further based at least on the processed third calibration measurements.

12. The computer-implemented method of any one of claims 3-11 when directly or indirectly depending on claim 2, wherein the at least one calibration model comprises:
a first calibration model for a first PFM state based on the processed first calibration measurements; and
a second calibration model for the second PFM state based on the processed second calibration measurements.

13. The computer-implemented method of claim 9 or of any one of claims 10 - 12 when directly or indirectly depending on claim 9, further comprising:
processing, by the at least one computing device, verification measurements taken during the one or more calibration positions by at least one optical sensor positioned to capture an image or video of at least a portion of the person or at least one motion tracking device arranged on the person; and
wherein the at least one calibration model is provided when the processed verification measurements are indicative of the person having been in each position of the one or more calibration positions.

14. A method comprising:
processing, by at least one computing device, first calibration measurements taken while a person has a first set of predetermined calibration positions such that, in each position of the first set, a pelvic floor muscle, PFM, of the person is in a first PFM state, the first calibration measurements being taken by a pressure sensor arranged inside the person adjacent to the PFM, the pressure sensor at least comprising an accelerometer, and the first calibration measurements being representative of both: pressure exerted on the pressure sensor by muscles of the person and an orientation of the pressure sensor each position of the first set;
processing, by the at least one computing device, second calibration measurements taken while the person has a second set of predetermined calibration positions such that, in each position of the second set, the PFM is in a second PFM state, the second calibration measurements being taken by the pressure sensor arranged inside the person adjacent to the PFM, and the second calibration measurements being representative of both: the pressure exerted on the pressure sensor by the muscles of the person and the orientation of the pressure sensor in each position of the second set; and
providing, by the at least one computing device, at least one calibration model that relates the pressure exerted on the pressure sensor by the muscles of the person to the orientations of the pressure sensor based on the processed first and second calibration measurements.

15. A system for tracking pelvic floor muscle (PFM) exercises, comprising:
an intra-body pressure sensor adapted for introduction into a vagina or an anus of a person; and
a computing device comprising a processor operative to:
process exercise measurements taken by a pressure sensor positioned adjacent to a pelvic floor muscle (PFM) of a person at least during performance of a physical exercise, wherein the exercise measurements are indicative of a pressure exerted on the pressure sensor and an orientation of the sensor; and
calibrate the pressure of the processed exercise measurements at least based on the orientation of the processed exercise measurements.
